# EUROPEAN PATENT SPECIFICATION

(11) **EP 4 444 184 B1**
(45) Date of publication and mention of the grant of the patent: **24.06.2026**
(21) Application number: 22822407.7
(22) Date of filing: 25.11.2022
(51) Int. Cl.: A61B 8/08

(54) **SYSTEM AND METHOD TO PREDICT NEED FOR USE OF A CONTRAST AGENT IN ULTRASOUND IMAGING**
SYSTEM UND VERFAHREN ZUR VORHERSAGE DER NOTWENDIGKEIT DER VERWENDUNG EINES KONTRASTMITTELS IN DER ULTRASCHALLBILDGEBUNG
SYSTÈME ET PROCÉDÉ POUR PRÉDIRE LE BESOIN D'UTILISER UN AGENT DE CONTRASTE DANS L'IMAGERIE ULTRASONORE

(30) Priority: 10.12.2021 US 202163288042 P
(43) Date of publication of application: 16.10.2024
(73) Proprietor: Koninklijke Philips N.V., 5656 AE Eindhoven (NL)
(72) Inventor: KRUECKER, Jochen, 5656 AG Eindhoven (NL); SADEGHI, Seyedali, 5656 AG Eindhoven (NL); ERRICO, Claudia, 5656 AG Eindhoven (NL); BHARAT, Shyam, 5656 AG Eindhoven (NL); PREVRHAL, Sven Peter, 5656AG Eindhoven (NL); XIE, Hua, 5656AG Eindhoven (NL)
(74) Representative: Philips Intellectual Property & Standards
(86) International application number: PCT/EP2022/083264
(87) International publication number: WO 2023/104547

(56) References cited:
- EP-A1- 3 571 997
- US-A1- 2018 071 452
- ELLENBERGER KATHERINE ET AL: "The Effect of Obesity on Echocardiographic Image Quality", HEART, LUNG AND CIRCULATION, ELSEVIER, AMSTERDAM, NL, vol. 31, no. 2, 7 August 2021 (2021-08-07), pages 207 - 215, XP086922048, ISSN: 1443-9506, [retrieved on 20210807], DOI: 10.1016/J.HLC.2021.06.525

## Description

### BACKGROUND

Ultrasound (US) imaging is ubiquitous in a variety of medical disciplines. For example, echocardiography is a useful tool that is widely adopted for examination of the functions of the heart. Often, an ultrasound enhancing agent (UEA) or "contrast agent" is used during echocardiography to improve the quality of the images, or to salvage non-diagnostic non-contrast images, or both.

While the usage of a contrast agent has increased steadily over time, its application in US imaging remains rather low with only approximately 4% to approximately 7% of US scans of the heart. The comparatively low use of contrast agents in echocardiography is not necessarily caused by a lack of need for the agent in a particular scan. Rather, the need to inject the contrast agent is often not realized until the US scan has begun. In such cases, the echocardiographic procedure must be halted to allow for the injection and delivery of a bolus of the contrast agent to the patient. Moreover, the sonographer is normally not trained to inject the contrast agent, and the US scan is delayed until a qualified clinician arrives to do the injection. Sometimes, in the interest of time, the sonographer moves ahead with the procedure without introducing the contrast agent, resulting in unsatisfactory US images.

So, in cases where there is a need for introduction of a contrast agent, there is either a delay in the procedure while the contrast agent is administered or the quality of the sonogram is substandard. As can be appreciated, either case is not optimal.

What is needed, therefore, is a method and system for administering a contrast agent in patients undergoing a US imaging procedure that overcomes at least the drawbacks of known methods and systems described above.

Document US2010098310 teaches segmentation of a scout image in order to estimate the required dose of contrast agent in an upcoming scan.

"The Effect of Obesity on Echocardiographic Image Quality", by Katherine Ellenberger et al., Heart, Lung and Circulation, Elsevier, Amsterdam, NL, vol. 31, no. 2, 7 August 2021, pp. 207-215 (XP086922048), evaluates the relationship between US image quality and body mass index and how this interplay translates into American Society of Echocardiography (ASE) recommendations for use of UEAs in real world cohort with a high population prevalence of obesity.

EP 3 571 997 A1 discloses a method for determining a patient weight and a body mass index of a patient. The method includes acquiring image data containing depth information of the patient, generating a surface model of the patient based upon the image data acquired, determining density information or X-ray attenuation information of at least part of the patient, and determining at least one of the total patient weight and the body mass index of the patient using the surface model generated and the density information or the X-ray attenuation information determined.

### SUMMARY

The invention is defined by the independent claims. Advantageous embodiments are provided in the dependent claims.

According to an aspect of the present disclosure, a method of performing ultrasound imaging is disclosed. The method comprises: retrieving prior imaging data for a patient; segmenting relevant structures from the prior imaging data; extracting quantitative parameters of the patient from the segmented relevant structures; and applying a trained predictive model to the extracted parameters to determine whether or not to apply a contrast agent to the patient.

According to another aspect of the present disclosure, a system for medical imaging is described. The system comprises: an ultrasound imaging device; a memory adapted to store: a trained predictive model comprising instructions; prior imaging data for a patient, or prior non-imaging data for the patient, or both; segmented relevant structures from the prior imaging data; and extracted quantitative parameters of the patient from the segmented relevant structures; and a controller, wherein the instructions, when executed by the controller, cause the controller, based on the quantitative parameters, to determine whether or not to apply a contrast agent to the patient.

According to another aspect of the present disclosure, a tangible, non-transitory computer readable medium that stores: instructions for a predictive model; prior imaging data for a patient, or prior non-imaging data for the patient, or both; segmented relevant structures from the prior imaging data; and extracted quantitative parameters of the patient from the segmented relevant structures, wherein the instructions, when executed by a controller, the instructions cause the controller to:determine whether or not to apply a contrast agent to the patient based on extracted quantitative parameters of the patient from segmented relevant structures.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a simplified block diagram of a system for imaging a portion of a body, according to a representative embodiment.
Fig. 2 is a simplified flow diagram showing elements of a method of determining whether or not a contrast agent should be administered according to a representative embodiment.
Fig. 3 is a simplified flow diagram showing a method of determining whether or not a contrast agent should be administered according to a representative embodiment.
Fig. 4 shows segmented image slices, a rib cage model and a subcutaneous fat segmentation in accordance with a representative embodiment.
Fig. 5 is a simplified flow chart of a method of training a predictive model in accordance with a representative embodiment.
Fig. 6 is a simplified flow chart of a method of determining whether or not a contrast agent should be administered according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

By the present teachings, the image quality of an upcoming US scan can be predicted. Based on this prediction, the determination can be made whether a contrast agent will be needed to improve the image quality of the upcoming scan. As described more fully below, this prediction is based on an automated analysis of the patient's prior non-US images (e.g., CT or MR). The prediction can also take into account other non-imaging data such as demographics, the patient's medical records including clinical data (e.g., age, body mass index (BMI)). Optionally, recent current US scan(s) may be used in the prediction as well.

The prior non-US scans of germane features of the patient's body (e.g., ribs and subcutaneous fat layer) are automatically segmented according to the present teachings, and used to extract parameters (e.g., rib spacing and fat layer thickness) that correlate with US image quality. As explained more fully below, these parameters are provided to a predictive model, which may be a regression model or a machine-learning model, to provide the prediction of whether or not a contrast agent should be used to provide satisfactory images from the US scan. For example, generally the less the rib spacing and the greater the fat layer thickness, the more likely it is that a contrast agent should be used. Notably, as used herein, the term "predictive model" is intended to encompass the trained regression models or the trained machine-learning models described herein, depending on the implementation.

This prediction can be provided on a display to alert the clinician performing the US scan and other members of the medical team (e.g., physicians, nurses) whether or not a contrast agent should be administered before the commencement of the US imaging procedure. Because the determination is made whether or not a contrast agent is needed for an upcoming scan, delays caused when the need for contrast agent is not determined until the imaging procedure has begun, or is foregone to avoid the delay caused by the need to provide the contrast agent to the patient during the imaging procedure, can be reduced, if not avoided. Beneficially, image quality can be improved compared to known techniques, and workflow arrangements to provide contrast agent before an imaging procedure begins without the delays attendant to prior techniques.

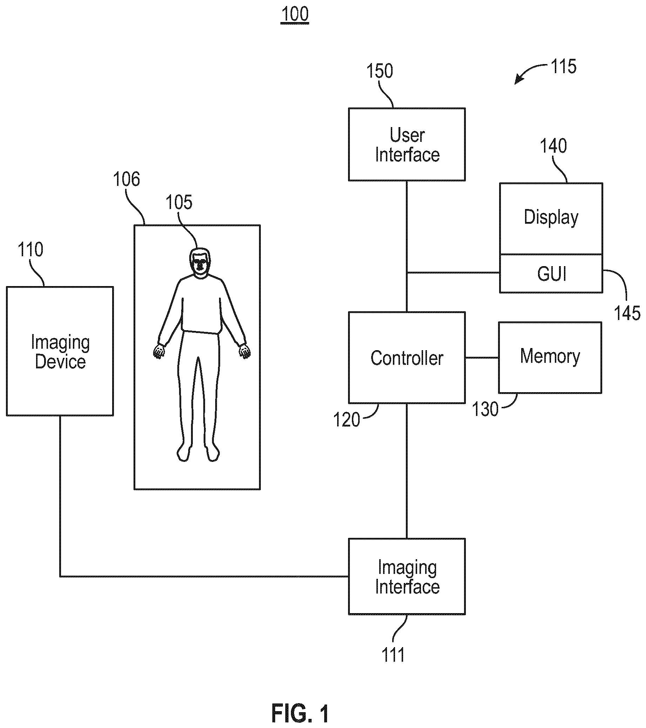

Fig. 1 is a simplified block diagram of an imaging system 100 for segmenting an image of a region of interest of a subject, according to a representative embodiment.

Referring to Fig. 1, the imaging system 100 includes an imaging device 110 and a computer system 115 for controlling imaging of a region of interest in a patient 105 on a table 106. The imaging device 110 may be any type of medical imaging device capable of providing an image scan of the region of interest in the patient 105. In accordance with representative embodiments described below, the imaging device is US imaging device or other imaging modality compatible with the methods and systems of the present teachings described herein. Notably, as will become clearer as the present description continues, US imaging is carried out with a selected US imaging device in accordance with a predictive model described below. Notably, and as will become clearer as the present description continues, the selected US imaging device is not typically a component of the imaging system 100. As such, US scans used in accordance with various representative embodiments are done separately from the imaging system 100.

The computer system 115 receives image data from the imaging device 110, and stores and processes the imaging data according to the embodiments discussed herein. The computer system 115 includes a controller 120, a memory 130 and a display 140.

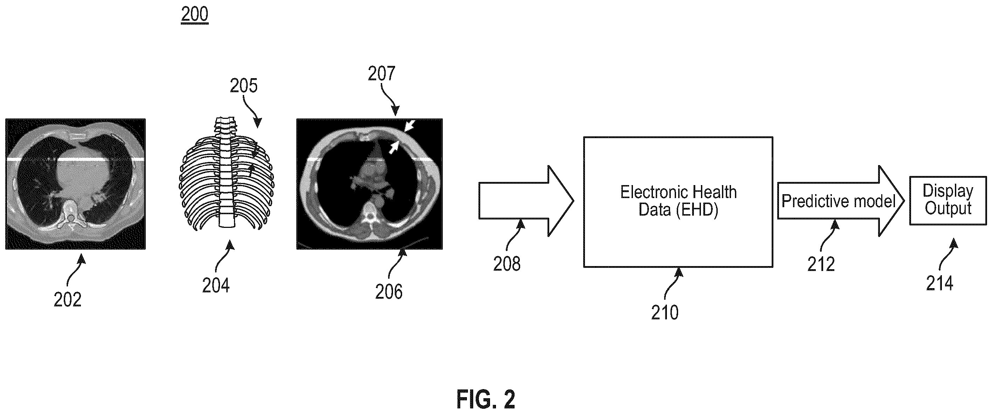

The controller 120 interfaces with the imaging device 110 through an imaging interface 111. The memory 130 stores instructions executable by the controller 120. When executed, and as described more fully below, the instructions cause the controller 120 to implement processes that include determining whether or not a contrast agent should be administered before the US procedure begins based on a predictive model based on the on various sources of data as described below with reference to FIGs. 2-5, for example. In addition, the controller 120 may implement additional operations based on executing instructions, such as instructing or otherwise communicating with another element of the computer system 115, including the display 140, to indicate whether or not a contrast agent should be administered prior to the US imaging procedure.

The controller 120 is representative of one or more processing devices, and is configured to execute software instructions to perform functions as described in the various embodiments herein. The controller 120 may be implemented by field programmable gate arrays (FPGAs), application specific integrated circuits (ASICs), a general purpose computer, a central processing unit, a computer processor, a microprocessor, a microcontroller, a state machine, programmable logic device, or combinations thereof, using any combination of hardware, software, firmware, hard-wired logic circuits, or combinations thereof. Additionally, any processing unit or processor herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems, such as in a cloud-based or other multi-site application. The term computing device should also be interpreted to include a collection or network of computing devices each including a processor or processors. Programs have software instructions performed by one or multiple processors that may be within the same computing device or which may be distributed across multiple computing devices.

The memory 130 may include a main memory and/or a static memory, where such memories may communicate with each other and the controller 120 via one or more buses. The memory 130 stores instructions used to implement some or all aspects of methods and processes described herein. The memory 130 may be implemented by any number, type and combination of random access memory (RAM) and read-only memory (ROM), for example, and may store various types of information, such as software algorithms, which serves as instructions, which when executed by a processor cause the processor to perform various steps and methods according to the present teachings. For example, in accordance with various representative embodiments, the memory 130 stores a trained predictive model comprising instructions; prior imaging data for a patient, or prior non-imaging data for the patient, or both; segmented relevant structures from the prior imaging data; and extracted quantitative parameters of the patient from the segmented relevant structures. The instructions, when executed by the processor, cause the processor, based on the quantitative parameters, to determine whether or not to administer a contrast agent to the patient.

The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium known in the art. The memory 130 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 130 may store software instructions and/or computer readable code that enable performance of various functions. The memory 130 may be secure and/or encrypted, or unsecure and/or unencrypted. More generally, and as will be appreciated from a review of the present disclosure, the inventive concepts also encompass a computer readable medium that stores instructions that cause a data processing system (such as the controller 120 of the imaging system 100) to execute the methods described herein. Examples of such media are noted above, and include non-transitory media such as computer memory devices that store information in a format that is readable by a computer or data processing system.

As described more fully below, the predictive models of the present teachings stored in the memory 130 are either a regression model or a machine-learning (AI) model, either of which is built in a training phase, using ground truth data from a population of N patients (N>=20) having undergone previous imaging scans of the portion of the body to be imaged in the upcoming ultrasound imaging procedure. In certain representative embodiments, the US imaging is of the heart (i.e., an echocardiogram). In this case, other image data of the heart (e.g., CT or MR image data), may be used as the ground truth data and provide the predictor variables of the trained model. Also, the imaging data from previous US imaging procedures of the particular portion of the body to be imaged can be included in the ground truth data of the population of patients.

As described in connection with representative embodiments below, the predictor variables are compiled by the processor using the image data of anatomical features of a portion of a body to be imaged using US. For example, when the upcoming procedure is an echocardiogram, the features to be segmented may include sternal ribs and the subcutaneous fat layer around the heart. These predictor variables are compiled by the processor to provide the imaging-derived parameters needed to run inferences on the trained predictive model. In addition, as alluded to above, and as described more fully below, non-imaging parameters may also be used in the building of the predictive model. These non-imaging parameters may include various data of the patient population from their medical records that are known to impact the decision whether or not to administer contrast agent based on previous imaging procedures. These non-imaging parameters may include, but are not limited to, demographic or clinical data that can result in the need for administration of a contrast agent such as age, a patient's body mass index (BMI) and presence of chronic obstructive pulmonary disorder (COPD). As will be appreciated by one of ordinary skill in the art, there is a correlation between BMI and the thickness of the subcutaneous fat layer that requires contrast when the BMI is greater than a thredshold. Similarly, the presence of COPD often requires administration of a contrast agent in an US imaging procedure of the heart.

Accordingly, the predictor variables (sometimes referred to as relevant parameters) comprising imaging and non-imaging parameters are assigned weights in the trained predictive model, which is applied to predictor variables to determine whether or not a contrast agent is needed. The response variable to the predictor variables is the "contrast need," using a scale of 0 to 1, where 0 means that no contrast is needed and 1 means that contrast is definitely needed. Ground truth for the "contrast need" is identified by expert annotation of the obtained echocardiography image. Again, the trained predictive model may be a known regression model including linear or non-linear regression, or logistic regression (after thresholding the response variable to either 0 or 1). Alternatively, the predictive model may be a known machine-learning model including random forest networks, Recurrent Neural Networks (RNNs) and other neural network based models, and computer programs, all of which are executable by the controller 120.

More generally, although not necessarily, the compiling of the imaging and non-imaging data to provide the parameters of the trained predictive model, and the training of the predictive model itself may be done using another processor and memory that are not necessarily part of the computer system 115 or the imaging system 100. In this case, after being trained, the predictive model may be stored as executable instructions in memory 130, for example, to be executed by a processor of the controller 120. Furthermore, updates to the predictive model may also be provided to the computer system 115 and stored in memory 130. Finally, and as will be apparent to one of ordinary skill in the art having the benefit of the present disclosure, according to a representative embodiment, the predictive model may be stored in a memory and executed by a processor that are not part of the computer system 115, but rather is connected to the imaging device 110 through an external link (e.g., a known type of internet connection). Just by way of illustration, the predictive model may be stored as executable instructions in a memory, and executed by a server that is remote from the imaging device 110. As such, the remote server may be configured to determine whether or not a contrast medium should be administered to the patient prior to the beginning of the imaging procedure and provide this determination to the clinician performing the imaging procedure (e.g., via an alert on the display 140). As alluded to above, this beneficially reduces delays in the imaging procedure caused by interruption of the imaging procedure for a contrast agent to be administered, or reduces the likelihood of unsatisfactory images that result from foregoing the administration of the contrast agent to avoid interruption of the imaging procedure.

The controller 120 illustratively includes or has access to the trained predictive model, which may be implemented as software. The trained predictive model may provide a deterministic decision based on a regression model, or a machined learning model (i.e., whether or not to administer a contrast agent). The predictive model (trained machine-learning model or trained regression model) may reside in any of various components in addition to or other than the controller 120, such as the memory 130, an external server, and/or a cloud, for example. When the predictive model is implemented in a cloud, such as at a data center, for example, the predictive model may be connected to the controller 120 via the internet using one or more wired and/or wireless connection(s). The predictive model may be connected to multiple different computers including the controller 120, so that the predictive models described below in connection with various representative embodiments are performed centrally based on and for a relatively large set of medical facilities and corresponding subjects at different locations. Alternatively, the predictive model and its training may be executed locally to the controller 120, such as at a single medical facility or in conjunction with a single imaging device 110.

The display 140 may be a monitor such as a computer monitor, a television, a liquid crystal display (LCD), a light emitting diode (LED) display, a flat panel display, a solid-state display, or a cathode ray tube (CRT) display, or an electronic whiteboard, for example. The display 140 may also provide a graphical user interface (GUI) 145 for displaying and receiving information to and from the user.

The interface 150 may include a user and/or network interface for providing information and data output by the controller 120 and/or the memory 130 to the user and/or for receiving information and data input by the user. That is, the interface 150 enables the user to enter data and to control or manipulate aspects of the processes described herein, and also enables the controller 120 to indicate the effects of the user's control or manipulation. The interface 150 may include one or more of ports, disk drives, wireless antennas, or other types of receiver circuitry. The interface 150 may further connect one or more user interfaces, such as a mouse, a keyboard, a mouse, a trackball, a joystick, a microphone, a video camera, a touchpad, a touchscreen, voice or gesture recognition captured by a microphone or video camera, for example.

As alluded to above, and as described in more detail below, in accordance with various representative embodiments, US imaging is used to provide images of a selected portion of a body with or without administration of a contrast agent, where the decision whether or not to administer the contrast agent is made based on a predictive model. The US methods and devices contemplated for imaging are illustratively, but not limited to contrast echocardiography, color Doppler imaging or 3D flow volumetry at specific locations/anatomical landmarks along the portion of the body being evaluated. It is emphasized that the application of the present teachings to the heart muscle is merely illustrative, and the present teachings are contemplated for use in other locations of the body where a contrast agent may be required.

Fig. 2 is a simplified flow diagram showing elements of a method of determining whether or not a contrast agent should be administered according to a representative embodiment. Various aspects and details of the method 200 are common to those described above, and may not be repeated in order to avoid obscuring the discussion of the present representative embodiment.

Prior images are collected from a medical database, such as discussed more fully below. A segmentation 202 of the rib cage and subcutaneous fat layer of a patient may be retrieved, and used to predict whether or not a contrast agent should be administered based on various parameters used in the predictive models of the present teachings. The segmentation 202 is garnered from previous images of the patient that generally include images from other modalities, including, but not limited to, magnetic resonance images (MRI) and computer tomography (CT) images. However, in certain applications the segmentation 202 may comprise previous US scans as well. Notably, segmentation on prior MRI or CT images may be beneficial because US images may not be available. For example, the current or upcoming US exam may be the first US exam in this patient). In addition, US has a limited field of view when compared to CT imaging and MRI, which can image the entire cross-section of the human body. As such, the proposed methods based on rib segmentation may not be feasible, or may not be as accurate, when based on prior US exams.

As noted above, the predictive model according to the present and other representative embodiments described herein are directed to an upcoming US scan of the heart (i.e., an echocardiogram). It is emphasized that this is merely an illustrative application of the present teachings, and is not intended to be limiting in any way. Rather, the method and system for determining whether a contrast agent should be administered to the patient is contemplated for use in other US image procedures that may incorporate the administering of a contrast agent to improve the quality of the scan. More generally, the method and system for determining whether a contrast agent should be administered before the imaging procedure begins may be adapted for use to enhance ultrasound imaging of blood perfusion in other organs (e.g. the liver, kidneys and bladder) and to measure blood flow rate in the heart and other organs.

A ribcage image 204 is provided from the previous imaging scans. As depicted in Fig. 2, the ribcage image 204 is used to determine the spacing 205 between the ribs. This parameter can be used in the predictive model to aid in the determination of whether a contrast agent should be administered to the patient.

Segmentation 206 of the subcutaneous fat layer surrounding the pleural cavity from previous imaging scans is also used in the predictive model to aid in the determination of whether a contrast agent should be administered to the patient. Specifically, a thickness 207 of the subcutaneous layer is determined from the previous imaging scan, since the thickness of this layer may indicate the need for a contrast agent to be administered.

At 208, the relevant parameters useful in predicting whether or not contrast agent should be administered are collected.

At 210, other data for the patient are gathered to provide other parameters useful to the predictive model to determine whether or not a contrast agent is indicated before the commencement of the US imaging procedure. These data are generally not imaging data, but are useful to predict the need the contrast agent in the upcoming procedure. Just by way of illustration of the particular data useful in the predictive model for an upcoming echocardiogram, these data include the existence or severity of comorbidities such as chronic obstructive pulmonary disease (COPD), body mass index (BMI) and demographic data. These data may be electronic health data (E.H.D.) extracted from the patient's electronic health record (E.H.R.), for example, by the imaging system 100 described above. Notably, and as described more fully below, these non-imaging data alone may be used to predict the need for a contrast agent in an upcoming procedure.

The imaging and non-imaging data of the patient can be provided by a digital imaging and communications in medicine (DICOM) header provided to the memory 130 of the imaging system 100 and accessed by the controller 120.

At 210, the imaging parameters or the non-imaging parameters, or both, are provided to the trained predictive model to determine whether or not a contrast agent, sometimes referred to colloquially as "contrast," should be used. As described more fully herein, the predictive model may be a trained regression model, such as a linear or non-linear regression model, or a logistic regression model.

Alternatively, the predictive model may be a trained machine-learning model such as a random forest model, or any convolutional neural network model. Examples of such models known to the ordinarily skilled artisan include, but are not limited to such as VGG-16, AlexNet, EfficientNet and SqueezeNet network architectures.

As alluded to above, and as described more fully below, the trained predictive models of the present teachings apply the current image and non-image parameters, with the weights of each parameter being based on its relative impact on the need for contrast. Based on these weighted parameters, the predictive model determines contrast should be applied if the average of the weights exceeds a certain threshold (e.g., 0.5 on a scale of 0 (no contrast) to 1(contrast needed)), or simply outputs 0 or 1.

As will be appreciated by one of ordinary skill in the art, in deep learning, which is a subfield of machine-learning, the inner parameters inside the computational model are organized into successively connected layers, where each layer produces increasingly meaningful output as input to the next layer, until the last layer which produces the final output.

Deep learning layers are typically implemented as so-called neural networks, that is, layers are comprised of respective sets of nodes, each representing an output value and a prescription on how to compute this output value from the set of output values of the previous layer's nodes. The prescription being a weighted sum of transformed output values of the previous layer's nodes, each node only needs to store the weights. The transformation function is the same for all nodes in a layer and is also called activation function. There are a limited number of activation functions that are used today. A particular way to set which previous layer's nodes provide input to a next layer's node is convolution. Networks based on this way are called convolutional networks.

Thus, in the learning phase or so-called training, for each example, the output of the final layer is computed. Outputs for all examples are compared with the desired outputs by way of the objective function. The output of the objective function, the so-called loss, is used as a feedback signal to adjust the weights such that the loss is reduced. The adjustment, i.e. which weights to change and by how much, is computed by the central algorithm of deep learning, so-called backpropagation, which is based on the fact that the weighted sums that connect the layer nodes are functions that have simple derivatives. The adjustment is iterated until the loss reaches a prescribed threshold or no longer changes significantly.

A deep-learning network thus can be stored (e.g., in memory 130) as a topology that describes the layers and activation functions and a (large) set of weights (simply values). A trained network is the same, only the weights are now fixed to particular values. Once the network is trained, it is put to use, that is, to predict output for new input for which the desired output is unknown.

At 212, the determination of whether or not a contrast agent should be administered is output is displayed, illustratively on the display 140. The output provides an indication of whether or not the contrast agent should be used for subsequent US imaging. Alternatively, using other known communications technologies, the output may be provided to clinicians, such as on mobile phones through a specialized mobile application ('app'). Continuing with the example of an echocardiogram, both cardiac CT/MRI and relevant echocardiography imaging data and non-imaging data from the pool of patients are used by the predictive model to determine the need (or not) for contrast agent in the upcoming US imaging procedure.

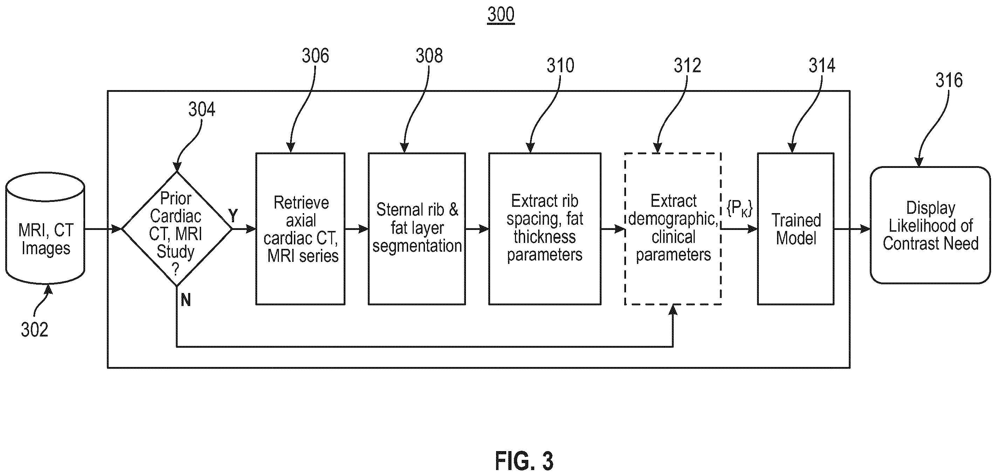

Fig. 3 is a simplified flow diagram showing a method 300 of determining whether or not a contrast agent should be administered according to a representative embodiment. The method 300 is contemplated for implementation using the imaging system 100. Various aspects and details of the method are common to those described above in connection with Figs. 1-2, and may not be repeated in order to avoid obscuring the discussion of the present representative embodiment.

At 302 the scan is scheduled for the patient, and using the medical record (MR) number prior scans (e.g., CT, MR) are retrieved and stored in memory 130.

At 304, the images are reviewed to determine if relevant scans are available. For example, if an echocardiogram is scheduled for the patient, the controller 120 searches for whether or not scans related to previous cardiac studies are available. In the event that imaging data from prior scans are not available, the method proceeds to 312 to determine whether a contrast agent is indicated based on non-imaging data.

At 306, the method comprises retrieving relevant scans for the particular anatomy to be imaged in the upcoming US imaging procedure. In accordance with a representative embodiment, the controller 120 identifies image series suitable for extracting information about the likelihood of administration of a contrast agent as alluded to above. In the present representative embodiment in which an echocardiogram is scheduled for the patient, these images may include ribs, or thickness of subcutaneous fat layers, or both. Illustratively, these features can be seen in standard axial CT/MRI image series, so the controller 120 can identify suitable series based on DICOM header information. Additional view classification can be performed using other known methods described to ensure that the ribs, subcutaneous fat layer, and other relevant aspects of the anatomy are in the field of view. By way of example, view classification for 306 may be as described in "Fast and Accurate View Classification of Echocardiograms Using Deep Learning," (Nature Partner Journals (NPJ)/Digital Medicine, March 21, 2018) by Madani, et al.

In addition, relevant scans may be identified based on the information about the scan protocol that was used to acquire the scans, which is stored as a data element in the DICOM header of the scans. As an example, if the determination about contrast need for an upcoming cardiac ultrasound scan is to be made, then CT/MRI scans previously acquired with a cardiac scan protocol will be retrieved. The retrieval may be performed by electronically requesting the scan in question from the Picture Archive and Communication System (PACS) of the hospital, for example.

When the relevant scans are available, the method 300 proceeds to 306, and when relevant scans are not available, the method 300 proceeds to 312, which is discussed below.

At 308, the retrieved scans are segmented by a known technique. Just by way of illustration, the segmentation of the retrieved MR/CT scans may be done according to the teachings of Klinder, et al.: "Automated Model-Based Rib Cage Segmentation and Labeling in CT Images" (Medical Image Computing and Computer-Assisted Intervention-MICCAI 2007. MICCAI 2007. Lecture Notes in Computer Science, vol. 4792. Springer, Berlin, Heidelberg).

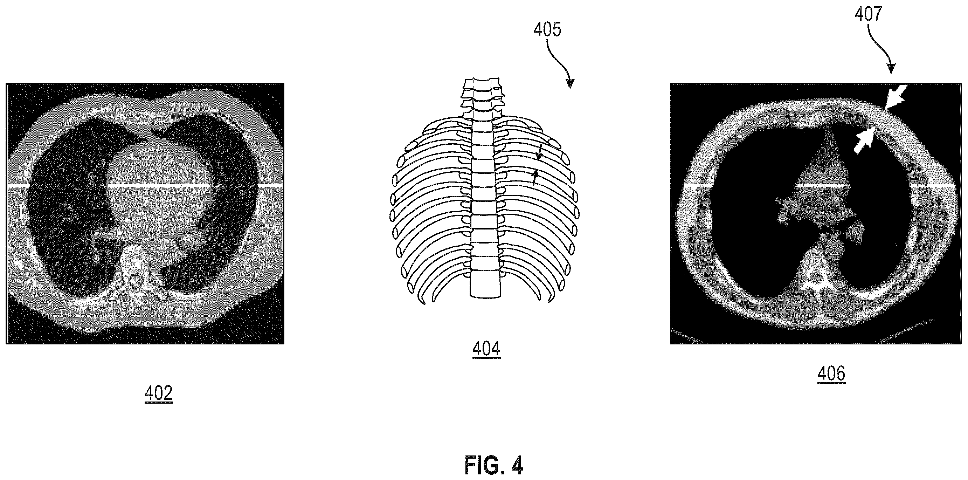

As will be appreciated by one of ordinary skill in the art, segmentation serves to extract and electronically quantify anatomical parts of the patient including for example, but not limited to, a surface of ribs and/or boundaries of fat layers that can subsequently be used to measure specific physical parameters (e.g., rib spacing, and subcutaneous fat thickness) that are known to be correlated with ultrasound image quality. Just by way of illustration, and as shown at 402 of Fig. 4, the features to be segmented automatically include, in the case of an echocardiogram, the sternal ribs and the subcutaneous fat layer around the heart. Of course, the segmentation of MR/CT scans of other parts of the anatomy would be carried out when the US imaging is for another portion of the body (e.g., the kidney).

At 310, the method 300 comprises extraction of relevant parameters for use in the trained predictive model to determine whether or not a contrast agent is indicated for the patient's upcoming US imaging procedure. As described more fully below, the relevant parameters extracted are identified by the trained predictive model as useful in the determination of whether or not a contrast agent is indicated for the upcoming scan for the patient. Again, in the representative embodiment in which the upcoming procedure is an echocardiogram, the extracted parameters may include quantitative rib spacing 405 of the ribs 404 and thickness 407 of subcutaneous fat layer 406 shown in Fig. 4.

At 312, the method continues with the addition of other relevant data that are generally not imaging data. As noted above, these data may be retrieved from the patient's EHRs or prior patient image DICOM header, and include relevant parameters for the particular US imaging procedure that are known to impact the need for administration of a contrast agent. Again, in the representative embodiment in which the upcoming US imaging procedure is an echocardiogram, these non-imaging data include, but are not limited to various comorbidities including a comparatively high BMI and COPD. Notably, just as the non-imaging data of the patient can cause the trained predictive model to recommend the need for a contrast agent, these data can be used to indicate that a contrast agent is not needed. So, the germane imaging data (e.g., rib spacing and subcutaneous fat layer thickness) and non-imaging data (e.g., BMI below a threshold and no evidence of COPD) can be used to predict that an upcoming US procedure does not require a contrast agent to be administered.

Furthermore, as noted above in the case when prior imaging data are not available, the method proceeds from 304 to 312.

At 314, the relevant parameters {pₖ} are provided to a trained predictive model. The trained predictive model is applied to the parameters to determine whether or not a contrast agent should be administered. As noted above, the trained predictive model may be stored as instructions in memory 130 and executed by the controller 120 to provide the decision of whether or not a contrast agent is indicated to the display 140. In accordance with a representative embodiment, the trained predictive model may be a known regression model or a machine-learning model. Based on all relevant parameters {pₖ} extracted from the current patient's imaging and non-imaging data the trained predictive model predicts the likelihood that a contrast agent (UEA) needs to be administered. The likelihood may be displayed at 316 as a numeric value (e.g. a percentage likelihood), combined with a text message highlighting the likely need (yes/no), and, in case of contrast need, the most important reason for the recommendation, as evident from the largest contributing variable(s) in the regression model (e.g. "Contrast Need Likelihood 75% - Subcutaneous Fat Layer 4.5cm, COPD"). Other combinations of displayed information, as well as graphical representations other than text and numeric values are also contemplated. These include, but are not limited to, color-coding of an exam according to contrast need (e.g. red = high likelihood of contrast need, green = low likelihood), a bar graph in which the bar size and/or color represents contrast need, a dial graph in which the orientation of the dial represents the likelihood of contrast need, or an audio signal that can be used by itself or in combination with a graphical representation to alert the user to need for administration of a contrast agent.

Moreover, if at 304, prior imaging data are not available and the method proceeds to 312, relevant parameters {pₖ} based on these non-imaging data are provided to the trained model at 314. Based on the relevant parameters {pₖ} based on these non-imaging data alone, the trained model determines whether a contrast agent should be administered.

The predictive (regression or machine-learning) model is built in a training phase described below, using imaging data and non-imaging data from a population of patients (N>=20) having undergone the upcoming US procedure. Continuing with the example of an echocardiogram, both cardiac CT/MRI and relevant echocardiography imaging data and non-imaging data from the pool of patients are used by the predictive model to determine the need (or not) for contrast agent in the upcoming US imaging procedure. Notably, these relevant data are the relevant parameters {pₖ} and may be referred to as predictor variables. The relevant parameters/predictor variables may be one or more of a variety of factors that impact the need for administration of a contrast agent. For example, rib spacing and subcutaneous fat thickness in an echocardiogram procedure are two such parameters. Notably, the smaller the rib spacing can result in the need for contrast agent. Similarly, and the thicker the subcutaneous fat layer is, the greater the likelihood that contrast agent will be needed. Accordingly, these relevant parameters are assigned a weight based in the algorithm of the predictive model based on their relative impact on whether or not a contrast agent is need for an upcoming imaging procedure. Accordingly, these relevant parameters impact the determination of whether or not a contrast agent should be administered before the beginning of the US imaging procedure.

The response variable (i.e., the output from the trained predictive model) will be the "contrast need," identified by expert annotation of the obtained echocardiography image, using a scale of 0 to 1. The predictive model can be linear or non-linear regression, logistic regression (after thresholding the response variable to either 0 or 1) models, or machine-learning models including random forests or convolutional neural networks.

The trained predictive model includes data from the population of patients that are germane to the particular upcoming US imaging procedure. As described below, the predictive model is trained using these data to determine the coefficients of the predictive model to provide a result that matches best the ground truth data of the population of patients. To this end, in accordance with one aspect of the present teachings, image data from the population of patients are reviewed by trained clinicians, such as radiologists. The clinician determines whether or not a contrast agent was indicated and should have been administered or not. The resultant determinations from the data reviewed by the trained clinician is stored as the ground truth data for the trained predictive model. Based on the stored data from the clinicians, the predictive model is trained to weight each of the image data and non-image data, which are the relevant parameters {pₖ}, so that the model predictions based on the image data and non-image data for the population of patients most closely match the ground truth data for this patient population.

As noted above, the trained predictive models of the present teachings may be non-linear, linear or logistic regression models, or machine-learning (AI) models that are known to those of ordinary skill in the art, such as Random Forest models, or convolutional neural network models such as VGG-16, AlexNet, EfficientNet or SqueezeNet. Regardless of the type of trained predictive models selected, the predictive model is trained so the coefficients (weights) of each of the parameters are optimized to predict whether the contrast agent is needed to most closely match the ground truth data.

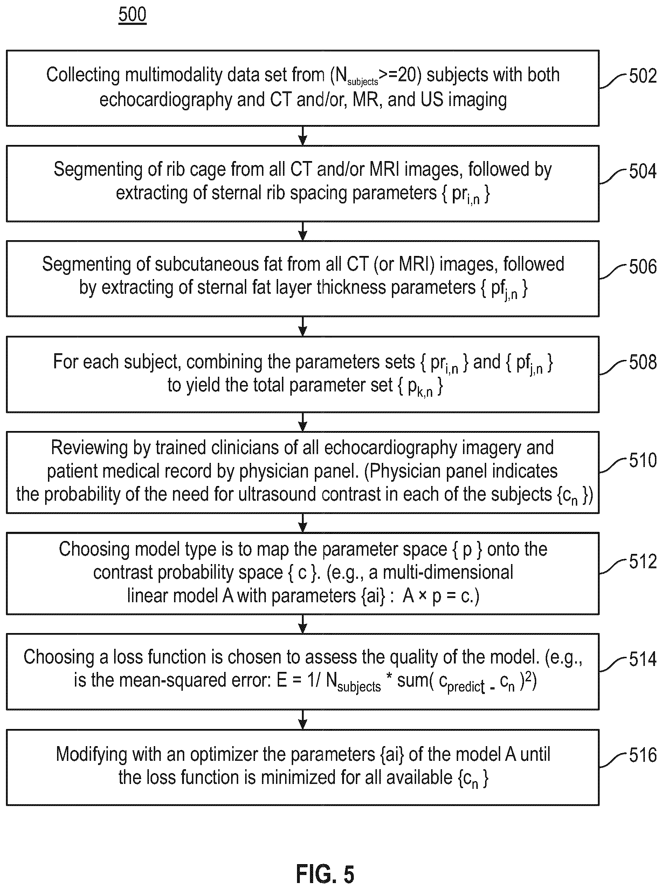

Fig. 5 is a simplified flow chart of a method 500 of training a predictive model in accordance with a representative embodiment. Notably, the method 500 is contemplated to be stored in memory 130 as executable instructions, which when executed by the controller 120 cause the controller 120 to provide a trained predictive model for further use by the imaging system 100. Various aspects and details of the method are common to those described above in connection with Figs. 1-4, and may not be repeated in order to avoid obscuring the discussion of the present representative embodiment.

For the present description, "{ }" indicates a set of parameter sets gathered from the population N, and indices i,j,k indicate parameter number, and index n indicates subject number.

At 502, the method 500 begins with collecting of the data set from the population N (N>=20) of patients who have had multimodality imaging. As describe above, this multimodality imaging may be CT and/or MR imaging, and US imaging. As noted above, these data are used to determine the coefficients (weights) of each of the relevant parameters {pₖ} to optimize the predictive model to mimic most effectively the ground truth data that include the determination of whether or not contrast agent is indicated for the upcoming scan.

At 504, the method 500 continues with the segmenting the rib cage from all scans (CT and/or MR) from the population of patients, followed by the extracting of the sternal rib spacing parameters {pr_{i,n}}. The segmentation of the rib cage from the scans of the population of patients is carried out in a manner described above, for example.

At 506, the method 500 continues with the segmenting of subcutaneous fat from all scans (CT, MR) from the population of patients, followed by extracting the subcutaneous fat layer thickness parameters{pf_{j,n}} for each patient. The segmentation of the subcutaneous fat layer from the scans of the population of patients is carried out in a manner described above, for example.

It is emphasized the sternal rib spacing parameters {pr_{i,n}}and the subcutaneous fat layer thickness parameters {pf_{j,n}} are intended to be illustrative, and more generally other anatomy-specific segmentation parameters that can be useful in the determination of whether a contrast medium should be administered based on the portion of the anatomy being imaged are contemplated by the present teachings. For example, the greater the subcutaneous fat layer thickness, the more likely a contrast medium should be administered.

At 508, the method 500 continues with the combining of the sternal rib spacing parameters {pr_{i,n}}and the subcutaneous fat layer thickness parameters {pf_{j,n}} to provide a total parameter set{p_{k,n}}for the entire population of patients. For example, the closer the sternal rib spacing, the more likely a contrast medium should be admisdtered. Again, these parameters are intended to be illustrative, and more generally other anatomy-specific segmentation parameters may be combined.

At 510, the method 500 continues with reviewing of all echocardiography imagery and patient medical records by trained clinicians. These trained clinicians include, but are not limited to a physician panel of experts (e.g., radiologists). Based on this review, the clinician panel indicates the probability of the need for ultrasound contrast in each of the subjects {cₙ }. Thus, at the end of the review, for any patient *n* of the N patients *(n =* 1..N), the set of parameters {p_{k,n}} for that patient can be paired with the expert-provided contrast probability cₙ for the same patient.

At 512, the method 500 continues with the selecting of a mathematical predictive model to map the parameter space {p} onto the contrast probability space {c_{predict}}. As noted above the predictive model may be a regression model or a machine language model. By way of illustration, the predictive model may be a multi-dimensional linear model (matrix A) with parameters {aᵢ}: A × p = c used to determine the need for administration of a contrast agent.

At 514, the method 500 continues with the selecting of a loss function to assess the quality of the predictive model. By way of illustration, this loss function may be a mean-squared error loss function: E = 1/ N * Σ (c_{predict} - cₙ )², where "Σ" represents the summation over the data for all training patients n = 1...N.

Finally, at 516, an optimizer is used to modify the parameters {aᵢ} of the predictive model A until the loss function is minimized for all available {cₙ }. In accordance with a representative embodiment, the optimizer may be one of a variety of optimizers including, but not limited to Gradient Descent Optimizer, Downhill Simplex Optimizer, or Adams optimizer.

Upon completion of the training of the predictive model, the trained predictive model is stored in memory 130 as instructions, which when executed by the controller 120, predict whether or not a contrast agent is needed for an upcoming imaging procedure (e.g., an echocardiogram) for a particular patient.

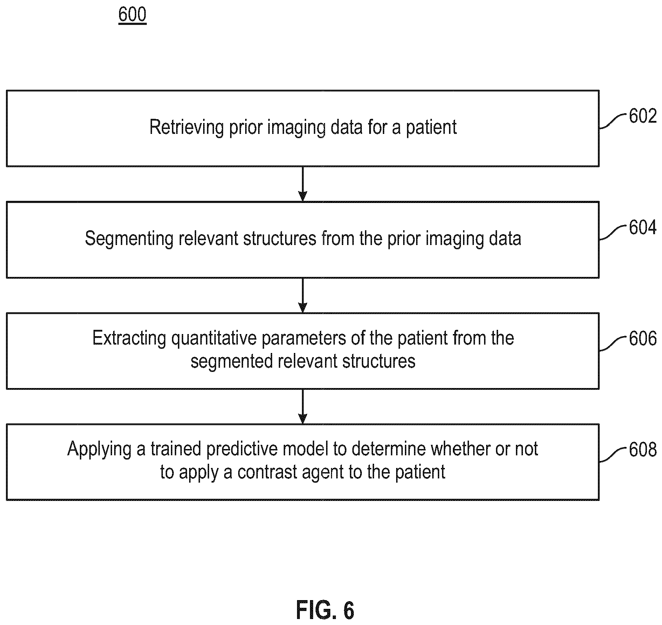

Fig. 6 is a simplified flow diagram showing a method 600 of imaging a portion of a body including training a computational predictive model, according to a representative embodiment.

Various aspects of the method and details of the flow diagram and the method 600 are common to those described above, and may not be repeated in order to avoid obscuring the discussion of the present representative embodiment. Most notably, the method 600 is contemplated for not only the method, but also for storing in a tangible, non-transitory computer readable medium that stores instructions (e.g., memory 130), which when executed by a processor (e.g., a processor of controller 120), cause the processor to carry out the method 600. Finally, while the method 600 is contemplated for use in echocardiography, the method 600 may be used in other systems where minimizing CM exposure, or radiation exposure of a patient, or both, is desired.

At 602, the method comprises: retrieving prior imaging data for a patient;

At 604, the method comprises segmenting relevant structures from the prior imaging data.

At 606, the method comprises extracting quantitative parameters of the patient from the segmented relevant structures.

At 608, the method 600 comprises applying a trained predictive model to determine whether or not to apply a contrast agent to the patient.

As will be appreciated by one of ordinary skill in the art having the benefit of the present disclosure, the systems and methods of the present teachings improve the function of an imaging system, such as the US imaging scanners described herein, and an improvement to the technical field of contrast-based medical imaging. For example, compared to known methods and systems, the need for administration of a contrast agent before a US imaging procedure can be predicted. This results in more efficient imaging at least because delays associated with halting the US imaging procedure so a contrast agent can be administered are reduced. Moreover, the quality of the US images generated may be improved at least because a patient who should have had a contrast agent, but did not, will be improved. Notably, the benefits are illustrative, and other advancements in the field of medical imaging will become apparent to one of ordinary skill in the art having the benefit of the present disclosure.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although methods, systems and components of performing US imaging, and especially to determine whether or not to apply a contrast agent to the patient before an imaging procedure is begun, have been described with reference to several exemplary embodiments, it is understood that the words that have been used are words of description and illustration, rather than words of limitation. Changes may be made within the purview of the appended claims, as presently stated and as amended, without departing from the scope of interventional procedure optimization in its aspects. Although developing adaptable predictive analytics has been described with reference to particular means, materials and embodiments, developing adaptable predictive analytics is not intended to be limited to the particulars disclosed; rather developing adaptable predictive analytics extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure as defined by the claims. Thus, to the maximum extent allowed by law, the scope of the present disclosure is to be determined by the broadest permissible interpretation of the following claims and shall not be restricted or limited by the foregoing detailed description.

## Claims

1. A method (600) of performing ultrasound imaging, the method (300) comprising:
retrieving prior imaging data (602) for a patient (105);
segmenting relevant structures from the prior imaging data (604);
extracting quantitative parameters of the patient (105) from the segmented relevant structures (606); and
applying a trained predictive model to the extracted quantitative parameters to determine whether or not to apply a contrast agent (608) to the patient (105) in an upcoming ultrasound scan.

2. The method (600) of claim 1, further comprising, before the retrieving:
determining when prior imaging data are available for the patient (105).

3. The method (600) of claim 2, wherein
when the prior imaging data are available for the patient, performing said steps of:
- retrieving the prior imaging data (602) for the patient (105),
- segmenting relevant structures from the prior imaging data (604), and
- extracting quantitative parameters of the patient (105) from the segmented relevant structures (606); and
when the prior imaging data are not available for the patient (105), instead performing the steps of:
- retrieving non-imaging data, and
- extracting the quantitative parameters from the non-imaging data.

4. The method (600) of claim 3, wherein the non-imaging data comprises demographic data, or clinical data from the patient (105), or both.

5. The method (600) of claim 1, wherein the prior imaging data comprises one or more of magnetic resonance imaging (MRI) data and computer tomography (CT) imaging data.

6. The method (600) of claim 1, wherein the segmenting further comprises applying a model containing anatomy-specific segmentation parameters.

7. The method (600) of claim 1, wherein the relevant structures comprise rib spacing (205), or a thickness (207) of subcutaneous fat, or both.

8. A system (100) for medical imaging, comprising:
an ultrasound imaging device (110);
a memory (130) adapted to store: a trained predictive model comprising instructions; prior imaging data for a patient (105), or prior non-imaging data for the patient (105), or both; segmented relevant structures from the prior imaging data; and extracted quantitative parameters of the patient (105) from the segmented relevant structures; and
a controller (120), wherein the instructions, when executed by the controller (120), cause the controller (120), based on the quantitative parameters, to determine whether or not to apply a contrast agent to the patient (105) in an upcoming ultrasound scan.

9. The system (100) of claim 8,
wherein the non-imaging data comprises demographic data, or clinical data from the patient (105), or both; and/or
wherein the prior imaging data comprises one or more of magnetic resonance imaging (MRI) data and computer tomography (CT) imaging data.

10. The system (100) of claim 8, wherein the ultrasound imaging device (110) comprises a cardiac imaging device.

11. A tangible, non-transitory computer readable medium that stores instructions for a trained predictive model; prior imaging data for a patient (105), or prior non-imaging data for the patient (105), or both; segmented relevant structures from the prior imaging data; and extracted quantitative parameters of the patient (105) from the segmented relevant structures, wherein the instructions, when executed by a controller (120), cause the controller (120) to:
determine whether or not to apply a contrast agent to the patient (105) in an upcoming ultrasound scan, based on extracted quantitative parameters of the patient (105) from segmented relevant structures.

12. The tangible, non-transitory computer readable medium of claim 11, wherein the segmented relevant structures are determined from prior imaging data of the patient (105).

13. The tangible, non-transitory computer readable medium of claim 11, wherein the non-imaging data comprises demographic data, or clinical data from the patient (105), or both.

14. The tangible, non-transitory computer readable medium of claim 11, wherein the prior imaging data comprises one or more of magnetic resonance imaging (MRI) data and computer tomography (CT) imaging data.

## Patentansprüche

1. Verfahren (600) zur Durchführung von Ultraschallbildgebung, wobei das Verfahren (300) Folgendes umfasst:
Abrufen von vorherigen Bildgebungsdaten (602) für einen Patienten (105);
Segmentieren relevanter Strukturen aus den vorherigen Bildgebungsdaten (604);
Extrahieren quantitativer Parameter des Patienten (105) aus den segmentierten relevanten Strukturen (606); und
Anwenden eines trainierten Vorhersagemodells auf die extrahierten quantitativen Parameter, um zu bestimmen, ob dem Patienten (105) bei einer bevorstehenden Ultraschalluntersuchung ein Kontrastmittel (608) verabreicht werden soll oder nicht.

2. Verfahren (600) nach Anspruch 1, ferner umfassend vor dem Abrufen:
Bestimmen, wann vorhergehende Bildgebungsdaten für den Patienten (105) verfügbar sind.

3. Verfahren (600) nach Anspruch 2, wobei
wenn die vorherigen Bildgebungsdaten des Patienten verfügbar sind, Durchführen folgender Schritte:
- Abrufen der vorherigen Bildgebungsdaten (602) für den Patienten (105),
- Segmentieren relevanter Strukturen aus den vorherigen Bildgebungsdaten (604), und
- Extrahieren quantitativer Parameter des Patienten (105) aus den segmentierten relevanten Strukturen (606); und
wenn die vorherigen Bildgebungsdaten für den Patienten (105) nicht verfügbar sind, Durchführen stattdessen folgender Schritte:
- Abrufen von Nicht-Bildgebungsdaten, und
- Extrahieren der quantitativen Parameter aus den Nicht-Bildgebungsdaten.

4. Verfahren (600) nach Anspruch 3, wobei die Nicht-Bildgebungsdaten demografische Daten oder klinische Daten des Patienten (105) oder beides umfassen.

5. Verfahren (600) nach Anspruch 1, wobei die vorherigen Bildgebungsdaten eines oder mehrere von Magnetresonanztomographie-Bildgebungsdaten (MRI-Bildgebungsdaten) oder Computertomographie-Bildgebungsdaten (CT-Bildgebungsdaten) umfassen.

6. Verfahren (600) nach Anspruch 1, wobei die Segmentierung ferner die Anwendung eines Modells umfasst, das anatomisch-spezifische Segmentierungsparameter enthält.

7. Verfahren (600) nach Anspruch 1, wobei die relevanten Strukturen einen Rippenabstand (205) oder eine Dicke (207) von subkutanem Fett oder beides umfassen.

8. System (100) zur medizinischen Bildgebung, umfassend:
eine Ultraschallbildgebungsvorrichtung (110);
einen Speicher (130), der Folgendes speichern kann: ein trainiertes Vorhersagemodell umfassend Anweisungen; vorhergehende Bildgebungsdaten eines Patienten (105) oder vorhergehende Nicht-Bildgebungsdaten des Patienten (105) oder beides; segmentierte relevante Strukturen aus den vorhergehenden Bildgebungsdaten; und extrahierte quantitative Parameter des Patienten (105) aus den segmentierten relevanten Strukturen; und
eine Steuereinheit (120), wobei die Anweisungen, wenn sie von der Steuereinheit (120) ausgeführt werden, die Steuereinheit veranlassen, auf der Grundlage der quantitativen Parameter zu bestimmen, ob dem Patienten (105) bei einem bevorstehenden Ultraschallscan ein Kontrastmittel verabreicht werden soll oder nicht.

9. System (100) nach Anspruch 8,
wobei die Nicht-Bildgebungsdaten demografische Daten oder klinische Daten des Patienten (105) oder beides umfassen; und/oder
wobei die vorhergehenden Bildgebungsdaten eines oder mehrere von Magnetresonanztomographie-Bildgebungsdaten (MRI-Bildgebungsdaten) und Computertomographie-Bildgebungsdaten (CT-Bildgebungsdaten) umfassen.

10. System (100) nach Anspruch 8, wobei die Ultraschallbildgebungsvorrichtung (110) eine Herz-Bildgebungsvorrichtung umfasst.

11. Greifbares, nichtflüchtiges, computerlesbares Medium, das Anweisungen für ein trainiertes Vorhersagemodell; vorhergehende Bildgebungsdaten eines Patienten (105) oder vorhergehende Nicht-Bildgebungsdaten des Patienten (105) oder beides; segmentierte relevante Strukturen aus den vorhergehenden Bildgebungsdaten; und extrahierte quantitative Parameter des Patienten (105) aus den segmentierten relevanten Strukturen speichert, wobei die Anweisungen, wenn sie von einer Steuereinheit (120) ausgeführt werden, die Steuereinheit (120) dazu veranlassen:
Bestimmen, ob dem Patienten (105) bei einem bevorstehenden Ultraschallscan ein Kontrastmittel verabreicht werden soll oder nicht, auf der Grundlage extrahierter quantitativer Parameter des Patienten (105) aus segmentierten relevanten Strukturen.

12. Greifbares, nichtflüchtiges, computerlesbares Medium nach Anspruch 11, wobei die segmentierten relevanten Strukturen aus vorhergehenden Bildgebungsdaten des Patienten (105) bestimmt werden.

13. Greifbares, nichtflüchtiges, computerlesbares Medium nach Anspruch 11, wobei die Nicht-Bildgebungsdaten demografische Daten oder klinische Daten des Patienten (105) oder beides umfassen.

14. Greifbares, nichtflüchtiges, computerlesbares Medium nach Anspruch 11, wobei die vorhergehenden Bildgebungsdaten eines oder mehrere von Magnetresonanztomographie-Bildgebungsdaten (MRI-Bildgebungsdaten) und Computertomographie-Bildgebungsdaten (CT-Bildgebungsdaten) umfassen.

## Revendications

1. Procédé (600) de réalisation d'imagerie par ultrasons, le procédé (300) comprenant :
la récupération des données d'imagerie antérieures (602) pour un patient (105) ;
la segmentation des structures pertinentes à partir des données d'imagerie précédentes (604) ;
l'extraction des paramètres quantitatifs du patient (105) à partir des structures pertinentes segmentées (606) ; et
l'application d'un modèle prédictif entraîné aux paramètres quantitatifs extraits pour déterminer s'il faut ou non appliquer un agent de contraste (608) au patient (105) lors d'une prochaine échographie.

2. Procédé (600) de la revendication 1, comprenant en outre, avant la récupération :
la détermination de quand des données d'imagerie antérieures sont disponibles pour le patient (105).

3. Procédé (600) de la revendication 2, dans lequel
lorsque les données d'imagerie antérieures sont disponibles pour le patient, effectuer les étapes suivantes :
- la récupération des données d'imagerie antérieures (602) pour le patient (105),
- la segmentation des structures pertinentes à partir des données d'imagerie précédentes (604), et
- l'extraction des paramètres quantitatifs du patient (105) à partir des structures pertinentes segmentées (606) ; et
lorsque les données d'imagerie antérieures ne sont pas disponibles pour le patient (105), effectuer à la place les étapes suivantes :
- la récupération des données non liées à l'imagerie, et
- l'extraction des paramètres quantitatifs à partir des données non liées à l'imagerie.

4. Procédé (600) de la revendication 3, dans lequel les données non liées à l'imagerie comprennent des données démographiques ou des données cliniques du patient (105), ou les deux.

5. Procédé (600) de la revendication 1, dans lequel les données d'imagerie antérieures comprennent une ou plusieurs données d'imagerie par résonance magnétique (IRM) et des données d'imagerie par tomodensitométrie (TDM).

6. Procédé (600) de la revendication 1, dans lequel la segmentation comprend en outre l'application d'un modèle contenant des paramètres de segmentation spécifiques à l'anatomie.

7. Procédé (600) de la revendication 1, dans lequel les structures pertinentes comprennent l'espacement des côtes (205), ou une épaisseur (207) de graisse sous-cutanée, ou les deux.

8. Système (100) d'imagerie médicale, comprenant :
un dispositif d'imagerie par ultrasons (110) ;
une mémoire (130) adaptée pour stocker : un modèle prédictif entraîné comprenant des instructions ; des données d'imagerie antérieures pour un patient (105), ou des données non liées à l'imagerie antérieures pour le patient (105), ou les deux ; des structures pertinentes segmentées à partir des données d'imagerie antérieures ; et des paramètres quantitatifs extraits du patient (105) à partir des structures pertinentes segmentées ; et
un dispositif de commande (120), dans lequel les instructions, lorsqu'elles sont exécutées par le dispositif de commande (120), amènent le dispositif de commande (120), sur la base des paramètres quantitatifs, à déterminer s'il faut ou non appliquer un agent de contraste au patient (105) lors d'une prochaine échographie.

9. Système (100) de la revendication 8,
dans lequel les données non liées à l'imagerie comprennent des données démographiques ou des données cliniques du patient (105), ou les deux ; et/ou
dans lequel les données d'imagerie antérieures comprennent une ou plusieurs données d'imagerie par résonance magnétique (IRM) et des données d'imagerie par tomodensitométrie (TDM).

10. Système (100) de la revendication 8, dans lequel le dispositif d'imagerie par ultrasons (110) comprend un dispositif d'imagerie cardiaque.

11. Support tangible, non transitoire et lisible par ordinateur, stockant des instructions d'un modèle prédictif entraîné ; des données d'imagerie antérieures d'un patient (105), ou des données non liées à l'imagerie antérieures du patient (105), ou les deux ; des structures pertinentes segmentées à partir des données d'imagerie antérieures ; et des paramètres quantitatifs extraits du patient (105) à partir des structures pertinentes segmentées, dans lequel les instructions, lorsqu'elles sont exécutées par un dispositif de commande (120), entraînent les actions suivantes dans le dispositif de commande (120) :
la détermination s'il faut ou non appliquer un agent de contraste au patient (105) lors d'une prochaine échographie, en fonction des paramètres quantitatifs extraits du patient (105) à partir des structures pertinentes segmentées.

12. Support tangible, non transitoire et lisible par ordinateur de la revendication 11, dans lequel les structures pertinentes segmentées sont déterminées à partir de données d'imagerie antérieures du patient (105).

13. Support tangible, non transitoire et lisible par ordinateur de la revendication 11, dans lequel les données non liées à l'imagerie comprennent des données démographiques ou des données cliniques du patient (105), ou les deux.

14. Support tangible, non transitoire et lisible par ordinateur de la revendication 11, dans lequel les données d'imagerie antérieures comprennent une ou plusieurs données d'imagerie par résonance magnétique (IRM) et des données d'imagerie par tomodensitométrie (TDM).
